# EUROPEAN PATENT SPECIFICATION

(11) **EP 1 397 170 B1**
(45) Date of publication and mention of the grant of the patent: **19.10.2005**
(21) Application number: 02737571.6
(22) Date of filing: 20.06.2002
(51) Int. Cl.: A61M 5/32

(54) **SAFETY SHIELD SYSTEM FOR PREFILLED SYRINGES**
SCHUTZHÜLSE FÜR VORGEFÜLLTE SPRITZEN
SYSTEME DE SECURITE POUR SERINGUES PREREMPLIES

(30) Priority: 20.06.2001 US 885815
(43) Date of publication of application: 17.03.2004
(73) Proprietor: Becton Dickinson and Company, Franklin Lakes, New Jersey 07417 (US)
(72) Inventor: GAGNIEUX, Samuel, F-38320 Eybens (FR); JANSEN, Hubert, 35041 Marburg-Michelbach (DE); BARRELLE, Laurent, F-38250 Saint Nizier du Moucherotte (FR)
(74) Representative: von Kreisler, Alek
(86) International application number: PCT/US2002/019715
(87) International publication number: WO 2003/000322

(56) References cited:
- EP-A- 0 966 983
- WO-A-01/60435
- US-A- 5 591 138

## Description

### BACKGROUND OF THE INVENTION

The field of the invention relates to shield systems for protecting against needle sticks, and syringes including such systems.

### Brief Description of the Related Art

Syringes are well known medical devices for administering medicaments to patients. Prefilled syringes are generally considered as those which are filled with a selected dosage of medicament by a pharmaceutical manufacturer for distribution to the end user. They are often comprised of a glass or plastic barrel which contains the medicament and a piston slidably mounted within the barrel. One end of the barrel includes a needle or the like affixed thereto or a connector for a needle assembly such as a Luer fitting. The other end of the syringe is open to allow the insertion of a plunger rod. The plunger rod allows the user to apply manual force to the piston, causing the medicament to be delivered through the needle or other piercing element.

The use of a sharp-pointed piercing element entails the risk of accidental needle stick. To avoid such accidents, many prior art hypodermic syringes have included rigid cylindrical safety shields telescoped over the syringe barrel. These shields can be moved between retracted positions where the needles are exposed for use, to extended positions where the needles are surrounded by the shields. U. S. Patent Nos. 4,425,120, 4,573,976, 4,850,994 and 4,923,447 disclose various shield systems for hypodermic syringes. The latter two patents disclose shields which are spring-actuated. It is ordinarily desirable to lock the needle shields in the protected positions, and a number of prior art designs provide for such locking. Some systems, such as those disclosed in U. S. Patent Nos. 5,201,708, 5,242,240 and 5,318,538 are designed to allow the shields to be retracted from their locked, extended positions.

A shield system for protecting the piercing element of a prefilled syringe is disclosed in European Publication No. EP 0 740 942 A1. The disclosed system includes a holder which is coupled to the flange of the syringe barrel, and a shield which is telescopically mounted to the holder. Two hands are required to operate this system.

EP 0 966 983 A1 to which the preamble of Claim 1 refers discloses a safety shield system for prefilled syringes including a syringe holder and a shield which is slidably coupled to the holder. The spring resiliently urges the shield from a retracted position to an extended position. Stop members are provided on the holder and shield for maintaining the shield in the retracted position. The syringe is slidably coupled to the holder, and extends within the shield. Axial movement of the syringe with respect to the holder causes disengagement of the stop members, allowing the spring to move the shield to the extended position. Detents are provided on the holder for maintaining the shield in the extended position.

US 5,591,138 discloses a protected needle assembly comprising a housing including a hub at a proximal end for mounting on at least one of the drug delivery system, a syringe and a blood receiving receptacle and a rigid tube. A needle is mounted on the hub and within the tube, extending beyond said tube. A sheath is disposed concentrically about said needle with one end extending beyond said needle in protective relation. The sheath is being movable longitudinally of said needle and said tube between an extended position covering over the needle and a retracted position exposing the needle. The assembly comprises a projection on one of the sheath and the tube, and a guide slot in the other of the sheath and the tube receiving the projection. The guide slot has a circumferentially directed portion at one end receiving the projection in the extended position of the sheath and an inclined portion at an opposite end directed to said one end to receive and retain the projection in the retracted position. Resilient means are provided for biasing the sheath from the retracted position to the extended position. Two hands are required to operate the assembly.

WO 01/60435 A1 discloses an automatically operable safety shield system for syringes comprising an inner holder into which a syringe may be inserted, an outer shield mounted outwards from the inner holder being axially movable between retracted and extended positions, a spring positioned between the inner holder and the outer shield, urging the outer shield to its extended position. The inner holder has an opening and, distally thereto, an indentation. The outer shield has a stop member engageable with the opening when the outer shield is in the retracted position. The stop member is engageable with the indentation when the outer shield is in the extended position. The outer shield may be released from its retracted position by action of a trigger positioned within the inner holder or by a protrusion on the syringe plunger.

### Summary of the invention

It is an object of the invention to provide a shield system for a syringe with easier and safer handling.

The shield system of the invention is defined by claim 1.

Accordingly the shield system comprises means for coupling a shield and a holder, said means for coupling are provided within the shield. A distal end of the means for coupling is coupled to a distal end portion of the shield. A first end of a spring which is coupled to the shield and to the coupling means and urging the shield towards an extended position is abutting the distal end portion of the shield.

The invention relates to a safety shield system for a syringe, and such a system as used in combination with an assembly capable of functioning as a syringe. In accordance with the preferred embodiment of the system, the user is able to cause the shielding of a needle by simply applying pressure to the plunger rod of the syringe following injection of the contents of the syringe barrel. The shield may accordingly be deployed automatically through the use of only one hand. As there is no need to place the hand near the needle for any purpose, the risk of needle stick injury is reduced.

In accordance with the objects of the invention, a medical device is provided which includes an automatically operable shield system mounted to a syringe barrel. The: system includes a holder which defines an enclosure. The syringe barrel extends at least partially, and preferably almost entirely, within the enclosure. The barrel is slidable within the holder. A shield is mounted to the holder, and positioned about at least a portion of the barrel. The shield is axially movable with respect to the holder between retracted and extended positions. It is intended to cover the needle tip when in the extended position. A spring engages the shield, and urges it towards the extended position. A stop member is positioned on the holder for releasably engaging the shield when the shield is in the retracted position. The force of the spring, by itself, is insufficient to cause disengagement of the shield and stop member. The barrel is operationally coupled to the shield such that sufficient axial movement of the barrel causes axial movement of the shield sufficient to cause disengagement of the shield and stop member. Such movement of the barrel is ordinarily caused by pressure on the plunger rod of the syringe following complete injection of the contents of the barrel. Upon disengagement of the shield and stop member, the spring causes the shield to move to the extended position.

The proximal end of the holder is preferably adapted to engage and retain the flange which may be present at the proximal end of the syringe barrel. The axial movement of the shield is preferably limited by a set of locking detents formed on the holder. Such movement could alternatively be limited by a tether connecting the holder and shield. The shield includes a distal end portion which is engaged by one end of the spring. The opposite end of the spring can bear against any suitable surface, such as the flange on the syringe barrel, if present, or preferably an abutment of an end fitting slidably positioned within the holder.

The shield system according to the invention is comprised of a holder, a shield, a spring and, preferably, an end fitting. The holder is adapted for receiving the barrel of a syringe. The shield is positioned within and connected to the holder, and is movable between retracted and extended positions. A spring engages the distal end portion of the shield and urges it towards the extended position. The holder includes a stop member which is engageable with the shield to maintain it in the retracted position. Sufficient axial movement of the shield causes disengagement of the stop member, allowing the spring to move the shield to the extended position. An end fitting is preferably incorporated in the system to maintain the position of the spring against the shield.

The shield system facilitates the safe use of prefilled syringes, though it can be adapted for other sharp-pointed medical devices, such as syringes filled just before use, as well. When employed with a syringe, the system allows the contents of the syringe to be expressed in a conventional manner. Continued, and preferably increased pressure on the plunger rod following injection causes the syringe barrel to move axially, thereby axially displacing the shield. Such displacement causes release of the stop member, and the spring to move the shield over the needle of the syringe. Protection against needle sticks is accordingly provided.

### BRIEF DESCRIPTION OF THE DRAWINGS

Fig. 1 is a top perspective view of a preferred embodiment the medical device according to the invention as assembled;
Figure 2 is an exploded, perspective view thereof;
Fig. 3 is a sectional elevation view thereof;
Fig. 4 is a sectional view thereof following actuation of the shield system of the device;
Fig. 5 is an enlarged sectional view of the proximal portion of the device prior to actuation of the shield system;
Fig. 6 is an enlarged sectional view showing a distal portion of the device prior to actuation of the shield system;
Fig. 7 is an enlarged sectional elevation view of a proximal portion of the device following actuation of the shield system; and
Fig. 8 is an enlarged sectional elevation view of the distal portion of the device following actuation of the shield system.

### DETAILED DESCRIPTION OF THE INVENTION

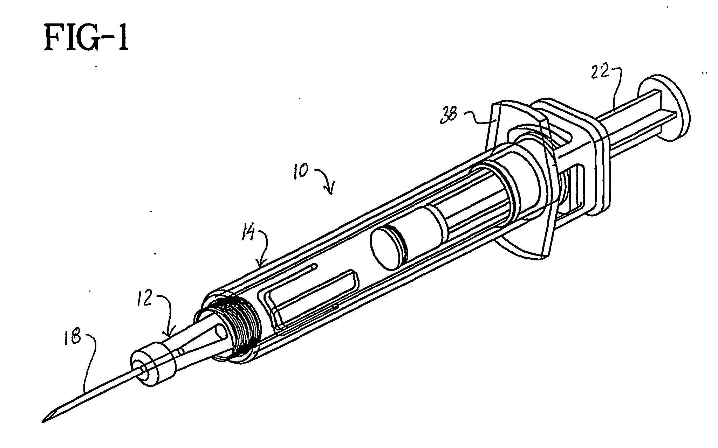

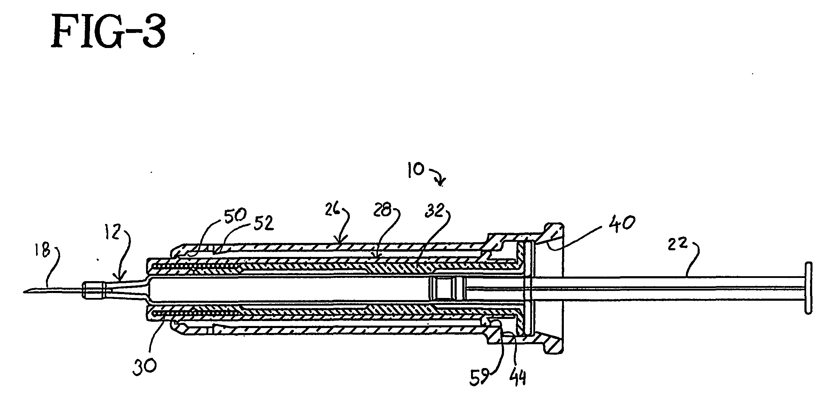

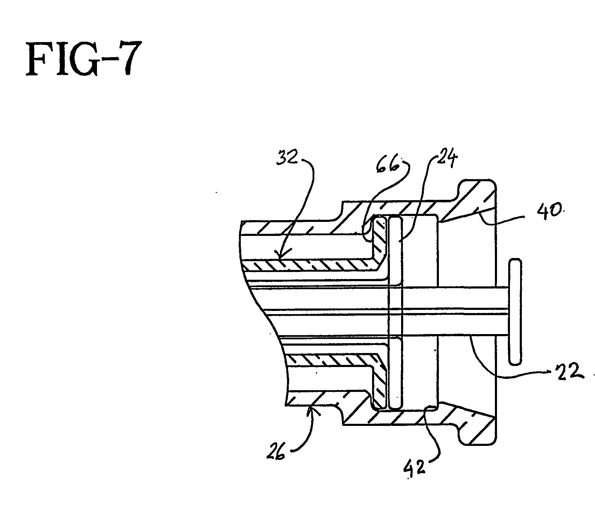

A medical device 10 for injecting a medicament into a patient is shown in Figures 1-8. The device comprises a prefillable syringe 12 and a shield system 14 coupled to the syringe, as shown in Fig. 1.

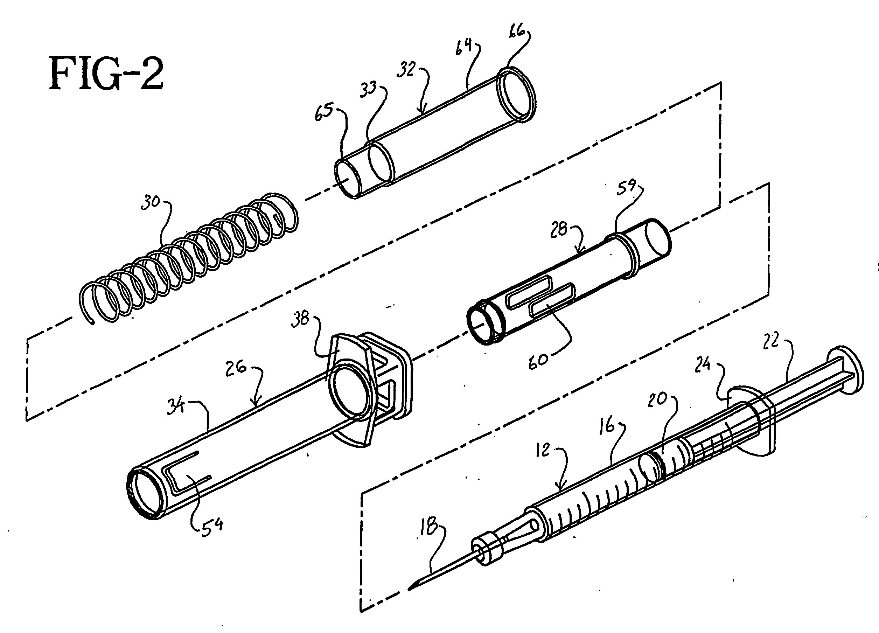

Syringes are ordinarily comprised of a generally cylindrical portion, known as a barrel, a needle or other piercing or connecting element secured to one end of the barrel, and a piston or stopper slidably positioned within the barrel. The needle may be removably secured to the barrel, but is more likely to be permanently secured to the barrel when the barrel is comprised of glass. Glass barrels are commonly used in prefillable syringes, and ordinarily contain a single dose of medication. Prefilled syringes made from plastic are also known to the art. The shield system 14 disclosed herein is employed in conjunction with a prefillable syringe including a barrel 16, a cannula such as a needle 18 permanently secured to the barrel, a piston 20 slidably positioned with the barrel, and a plunger rod 22 engageable with the piston as shown in Fig. 2. The syringe barrel 16 includes a radially outwardly extending flange 24, which is used to couple the syringe to the shield system.

The shield system 14 according to the invention includes a holder 26, a shield 28 coupled to the holder, and a spring 30. It also preferably includes a holder end fitting 32 which engages one end of the spring. With the exception of the spring, all of the components of the system are made from a semi-rigid plastic material such as polypropylene. The spring is preferably a metal coil spring.

The holder 26 is preferably comprised of an elongate, generally cylindrical body 34 which defines a generally cylindrical enclosure. The holder has proximal and distal open ends which provide access to the enclosure. A flange 38 extends radially outwardly from the holder body near the proximal open end thereof. The flange and body of the holder are designed for easy handling as an injection is made. Only one hand should be required for injection.

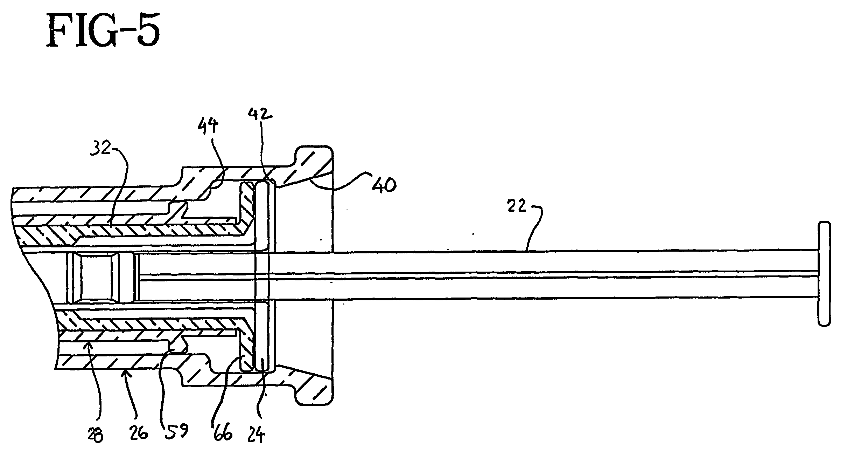

The inner surface of the holder includes a frustoconical portion 40 adjoining the proximal open end, as best shown in Fig. 5. A first abutment surface 42 is formed at the inner end of this surface. A second abutment surface 44 is formed by the holder body in opposing relation to the first abutment surface. As described below, the axial spacing between these surfaces corresponds, though may not be equal to the axial distance which the syringe can move with respect to the holder. The inner diameter of the holder, measured at the abutment surfaces, is smaller than the distance between the edges of the syringe flange 24. Accordingly, once the syringe is inserted far enough into the holder that the flange 24 is between abutment surfaces 42, 44, it is slidably coupled to the holder. The spring 30 urges the syringe flange into engagement with the first abutment surface 42.

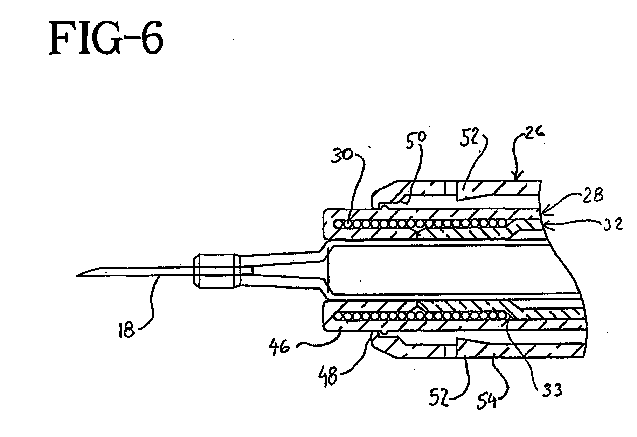

A generally annular stop member 48 is provided on the holder in the form of an inwardly extending protrusion, as shown in Fig. 6. Alternatively, a series of discrete protrusions (not shown) may be employed. The stop member is positioned at or near the distal end of the holder, and is preferably integral with the holder. A slot or other equivalent feature (not shown) may be provided in the holder near the stop member to provide flexibility therein.

A first detent 50 is provided at or near the first open end of the holder. The detent 50 may be in the form of an annular shoulder formed in the holder. Alternatively, the detent may be in the form of radially inwardly extending projections (not shown). The exact structure of the detent is not considered to be critical so long as it is capable of functioning satisfactorily in the manner described hereafter.

A second pair of detents 52 are provided on the holder, and are axially spaced from the first detent as shown in Fig. 6. Each of these detents 52 is formed on an axially extending arm 54 which is integral with the holder body 34 and pivotable with respect thereto. The end surface of each detent facing the distal open end of the holder is substantially perpendicular to the longitudinal axis of the holder. An inclined end surface is provided on the opposite side of each detent, and faces the proximal open end.

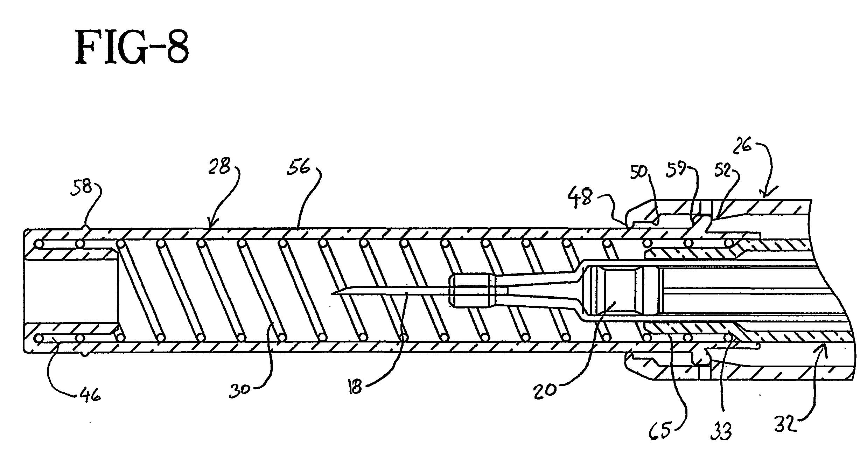

The shield 28 is comprised of a substantially cylindrical body 56 as shown in Fig. 8. It is preferably small enough in diameter to be positioned within the holder, and large enough to fit over the barrel 16 of the syringe and the end fitting 32. A stop member 58 in the form of a radially outwardly extending collar is formed on the body 56 of the shield near the distal end thereof. A second radially outwardly extending collar is formed on the body 56 towards the proximal end, and defines a further stop member 59. The second collar is larger in diameter than the first. Openings 60 in the shield provide flexibility for the shield body.

The spring 30 is sized to fit within the shield and over a reduced diameter portion of the end fitting. As shown most clearly in Figs. 6 and 8, a spring retainer in the form of an annular enclosure 46 is defined by the distal end of the shield. One end of the spring is positioned within this enclosure, and bears against the end wall of the shield. The other end of the spring abuts a shoulder portion 33 of the end fitting.

The spring may be used to cause the shield to move axially upon axial movement of the syringe barrel if it is fully compressed when the shield is in the retracted position. Direct engagement of the end fitting 32 and shield, as provided in the preferred embodiment, would be unnecessary in such an arrangement.

The end fitting 32 includes a generally cylindrical body 64 having a distal portion 65 of reduced diameter, as shown in Fig. 8. One end of the spring 30 fits over the reduced diameter portion, an abuts the shoulder 33. An annular wall 66 is provided at one end of the cylindrical body 64, and is preferably integral therewith. This wall extends radially outwardly with respect to the cylindrical body 64. The wall is adapted to engage the first abutment surface 42, so that it can be snapped behind the frustoconical portion at the proximal open end of the holder. The end fitting maintains the spring 30 in position within the holder, thereby allowing the shield system to be manufactured as an assembly which does not include the syringe. Axial movement of the syringe causes corresponding axial movement of the end fitting, which in turn urges the shield 28 in the distal direction.

The assembly and use of this preferred embodiment of the invention shall now be described. The shield 28 is slidably mounted to the holder by inserting it through the proximal open end thereof. The engagement of the stop members 48, 58 limits such insertion. The spring is inserted through the proximal open end of the holder, and within the shield until one end is positioned within the enclosure 46 and abuts the end wall of the shield. As a final step prior to providing the shield system to the end user, the end fitting 32 is inserted within the holder and into the other end of the spring. The spring is substantially compressed during this step. Once the annular wall 66 snaps over the frustoconical portion 42 of the holder, assembly of the shield system is complete. The shield is resiliently urged towards the distal end of the holder while the end fitting is urged towards the proximal end thereof. Neither element can move due to the engagement of the stop members 48, 58, and the annular wall 66 with the first abutment surface 42, respectively. The force of the spring 30 is insufficient to cause the disengagement of these members. The shield system may be provided to end users or pharmaceutical manufacturers in this form, ready for the insertion of a prefilled syringe.

The shield system 14 receives a syringe of appropriate size through the proximal open end of the holder. The syringe is in sliding engagement with the inner surface of the end fitting. The system as shown is designed for receiving a syringe including a flange. The syringe is inserted into the shield until the flange 24 snaps behind the first abutment surface 42. The end fitting 32 is displaced slightly during this procedure. As the needle of the syringe is ordinarily protected by a cover at this time, it may be safely coupled to the shield system.

The force required to disengage the stop members 48,58 should be greater than the force required to expel the contents of the syringe barrel. The plunger rod is employed to move the piston 20 down the syringe barrel until the contents of the barrel have been completely expelled. (The cover is, of course, removed prior to injection.) The contents of the barrel of a prefillable syringe ordinarily correspond to a single dose of the prescribed medicament.

Following removal of the needle 18 from the patient, the user applies a greater force to the plunger rod than that applied during injection. Such force causes axial displacement of the end fitting, the spring and the shield with respect to the holder. The distance between the annular wall 66 of the end fitting (or the flange 24) and the second abutment surface 44 is sufficient to allow the second stop member 58 to move far enough axially to where its retention by the first stop member 48 is overcome by the force of the spring. In the preferred embodiment, this is accomplished as the stop members slide past each other. The first stop member 48 may also be displaced radially as such sliding occurs if insufficient flexibility of the holder body is provided.

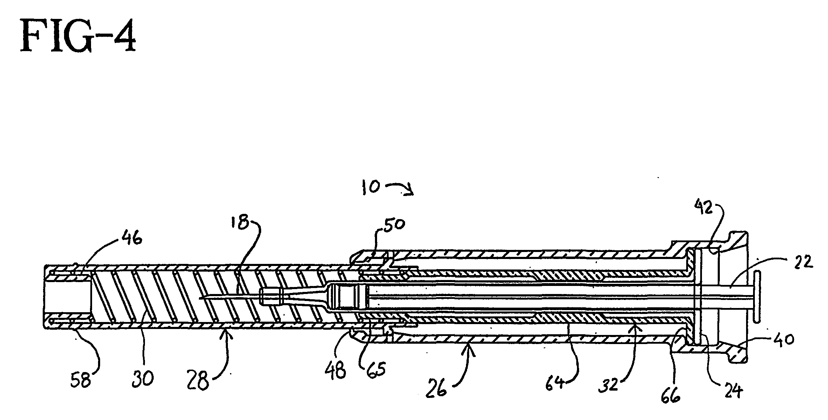

Once the stop members 48, 58 are disengaged, the spring 30 expands rapidly, causing the shield to slide axially with respect to the holder and syringe barrel to the position shown in Fig. 4. The stop member 59 moves past the second detents 52, causing them to deflect radially outwardly and then inwardly to their original positions. It finally engages the first detent 50. Upon such engagement, the needle 18 is entirely and permanently covered by the shield, as shown in Figs. 4 and 8. The shield cannot be retracted sufficiently to expose the needle tip due to the engagement of the stop member 59 with the second detents. It cannot be removed from the holder as the stop member 59 cannot move past the first detent 50.

The above-described procedure is particularly safe as it can be accomplished using only one hand. No second hand is required to push a button or use any other actuating member to release the spring. The risk of accidental actuation of the shield through inadvertent contact with an actuating button is eliminated. Moreover, a one-handed system is simpler for most people to use. It is readily apparent that the shield system can be adapted for use with syringes of various shapes and sizes without major modification.

The deployment of a shield in response to the axial displacement of a syringe barrel with respect to a holder is a safe and effective way of protecting against needle sticks. The preferred embodiment of the invention, as described above, provides advantages for the user as well as the manufacturer. The components are relatively easy to manufacture and assemble. It will be appreciated, however, that modifications can be made without changing the basic mode of operation of the device. For example, the stop member 58, rather than being in the form of a collar, can simply be the end of the shield. The stop member 48 and first detent 50 could be combined in a single element. It would also be possible to form the annular enclosure 46 at a different axial position. Connection of the spring to the end fitting and shield would obviate the need for the first detents. The spring would then function as a tether which would limit the axial movement of the shield. The dimensions of each component of the medical device are determined by the specific uses(s) for which it is designed.

It will be appreciated and understood by those skilled in the art that further and additional revisions to the invention may be devised without departing from the scope of the appended claims, the invention not being limited to the specific embodiments shown.

## Claims

1. A shield system (14) for a syringe (12) having a barrel (16) having an open proximal end and a distal end having a needle (18) secured thereto, said system (14) comprising:
a holder (26) comprising an elongate body (34), an elongate enclosure defined by said body, and proximal and distal open ends, the syringe for being held within said holder and for being axially movable therein;
an elongate shield (28) connected to said holder (26) and having a distal end portion, said shield (28) being axially displaceable with respect to said holder (26), said shield (28) being positioned at least partially within said enclosure and being axially slidable within said enclosure from a retracted position, wherein in use the needle (18) is not contained within said shield (28), to an extended position wherein in use the needle (18) is contained within said shield (28);
a fitting element (32) having a proximal end for coupling to the open proximal end of the syringe barrel (16), and a distal end;
a stop member (48) provided on said holder (26) and engageable with said shield (28), said stop member (48) being positioned to maintain said shield (28) in the retracted position and release said shield (28) upon sufficient axial displacement of said shield (28); and
a spring (30) coupled to said shield (28) and said fitting element (32) and urging said shield (28) towards an extended position, said spring (30) including a first end and a second end abutting said fitting element (32),
**characterized in that**
said fitting element (32) is positioned within said shield (28), and is for fitting said shield (28) onto said syringe barrel (16),
said distal end of said fitting element (32) is coupled to said distal end portion of said shield (28), and
said first end of said spring (30) is abutting said distal end portion of said shield (28).

2. A system (14) as described in Claim 1 wherein said coupling means comprises an elongate end fitting (32) slidably coupled to said holder (26), said spring (30) being coupled to said shield (28) by said end fitting (32).

3. A system (14) as described in Claim 2 wherein said shield (28) is slidably mounted upon said end fitting (32).

4. A system (14) as described in Claim 3 wherein said shield (28) includes a spring retainer (46) near the distal opening thereof, said spring (30) having an end retained by said spring retainer (46).

5. A system (14) as described in Claim 1 wherein said coupling means comprises an elongate end fitting (32) slidably mounted to said holder (26), said shield (28) being slidably mounted upon said end fitting (32), said spring (30) having a first end engaging said end fitting (32) and a second end engaging said shield (28).

6. A system (14) as described in Claim 5 wherein said first end of said spring (30) extends over a distal end of said end fitting (32).

7. A system (14) as described in Claim 6 wherein said shield (28) includes a spring retainer (46) near the distal opening thereof, said second end of said spring (30) being retained by said spring retainer (46).

8. A system (14) as described in Claim 4 wherein said spring retainer (46) defines an end of said shield (28).

9. A system (14) as described in Claim 8 wherein said end fitting (32) includes a shoulder (33) engaging said second end of said spring.

10. A system (14) as described in Claim 1 including a retainer at a proximal end portion of said holder (26), said holder (26) including a distal end portion including a stop member (48) engageable with said shield (28) when said shield (28) is in its retracted position, the force of said spring (30) being insufficient to cause disengagement of said shield (28) and stop member (48).

11. A system (14) as described in Claim 1 wherein said body of said holder (26) includes a first detent (50) and a second detent (52), said second detent (52) being axially spaced from said first detent (50), said shield (28) including a projection (58) positionable between said first (50) and second (52) detents when said shield (28) is in the extended position.

12. A system (14) as described in one of Claims 1-11, comprising a syringe (12) including a barrel (16) having an open proximal end and a distal end having a needle (18) secured thereto, said syringe (12) being coupled to said holder (26) and slidably positioned within an enclosure defined by said body, said syringe (12) being axially slidable within said enclosure.

13. A syringe (12) in combination with a shield system (14) according to any one of Claims 1-11, said syringe (12) including a barrel (16) having an open proximal end and a distal end having a needle (18) secured thereto, said syringe (12) being coupled to said holder (26) and slidably positioned within an enclosure defined by said body, said syringe (12) being axially slidable within said enclosure.

## Patentansprüche

1. Schutzsystem (14) für eine Spritze (12) mit einem Zylinder (16), der ein offenes proximales Ende und ein distales Ende, an welchem eine Nadel (18) angebracht ist, aufweist, wobei das System (14) umfaßt:
einen Halter (26) mit einem länglichen Körper (34), einer durch den Körper gebildeten länglichen Hülle, und einem proximalen und einem distalen offenen Ende, wobei die Spritze in dem Halter aufnehmbar und in diesem axial bewegbar ist;
einen länglichen Schutz (28), der mit dem Halter (26) verbunden ist und einen distalen Endbereich aufweist, wobei der Schutz (28) in bezug auf den Halter (26) axial verschiebbar ist, wobei der Schutz (28) zumindest teilweise in der Hülle angeordnet ist und in der Hülle aus einer eingefahrenen Position, in welcher die Nadel (18) im Gebrauch nicht in dem Schutz (28) enthalten ist, in eine ausgefahrene Position, in der die Nadel (18) im Gebrauch in dem Schutz (28) enthalten ist, verschiebbar ist;
ein Aufsetzelement (32) mit einem proximalen Ende zum Verbinden mit dem offenen proximalen Ende des Spritzenzylinders (16), und einem distalen Ende;
ein an dem Halter (26) vorgesehenes Anschlagteil (48), das in Eingriff mit dem Schutz (28) bringbar ist, wobei das Anschlagteil (48) derart positioniert ist, daß es den Schutz (28) in der eingefahrenen Position hält und den Schutz (28) bei einer ausreichenden axialen Verschiebung des Schutzes (28) freigibt; und
eine mit dem Schutz (28) und dem Aufsetzelement (32) verbundene Feder (30), welche den Schutz (28) in Richtung der ausgefahrenen Position drückt, wobei die Feder (30) ein erstes Ende und ein an dem Aufsetzelement (32) anliegendes zweites Ende aufweist,
**dadurch gekennzeichnet, daß**
das Aufsetzelement (32) in dem Schutz (28) angeordnet ist und dem Aufsetzen des Schutzes (28) auf den Spritzenzylinder (16) dient,
das distale Ende des Aufsetzelements (32) mit dem distalen Endbereich des Schutzes (28) verbunden ist, und
das erste Ende der Feder (30) an dem distalen Endbereich des Schutzes (28) anliegt.

2. System (14) nach Anspruch 1, bei dem die Verbindungseinrichtung ein längliches Endaufsetzelement (32) aufweist, das gleitend verschiebbar mit dem Halter (26) verbunden ist, wobei die Feder (30) durch das Endaufsetzelement (32) mit dem Schutz (28) verbunden ist.

3. System (14) nach Anspruch 2, bei dem der Schutz (28) gleitend verschiebbar auf dem Endaufsetzelement (32) angebracht ist.

4. System (14) nach Anspruch 3, bei dem der Schutz (28) eine Federrückhalteeinrichtung (46) nahe der distalen Öffnung derselben aufweist, wobei die Feder (30) ein von der Federrückhalteeinrichtung (46) gehaltenes Ende aufweist.

5. System (14) nach Anspruch 1, bei dem die Verbindungseinrichtung ein langgestrecktes Endaufsetzelement (32) aufweist, das gleitend verschiebbar an dem Halter (26) angebracht ist, wobei der Schutz (28) gleitend verschiebbar auf dem Endaufsetzelement (32) angebracht ist, wobei die Feder (30) ein erstes an dem Endaufsetzelement (32) angreifendes erstes Ende und ein an dem Schutz (28) angreifendes zweites Ende aufweist.

6. System (14) nach Anspruch 5, bei dem das erste Ende der Feder (30) sich über ein distales Ende des Endaufsetzelements (32) erstreckt.

7. System (14) nach Anspruch 6, bei dem der Schutz (28) eine Federrückhalteeinrichtung (46) nahe der distalen Öffnung desselben aufweist, wobei das zweite Ende der Feder (30) von der Federrückhalteeinrichtung (46) gehalten ist.

8. System (14) nach Anspruch 4, bei dem die Federrückhalteeinrichtung (46) ein Ende des Schutzes (28) bildet.

9. System (14) nach Anspruch 8, bei dem die Endaufsetzeinrichtung (32) eine an dem zweiten Ende der Feder angreifende Schulter (33) aufweist.

10. System (14) nach Anspruch 1, mit einer Rückhalteeinrichtung am proximalen Endbereich des Halters (26), wobei der Halter (26) einen distalen Endbereich mit einem Anschlagteil (48) aufweist, das in Eingriff mit dem Schutz (28) bringbar ist, wenn der Schutz (28) sich in der Rückzugsposition befindet, wobei die Kraft der Feder (30) nicht ausreicht, um ein Trennen des Schutzes (28) und des Anschlagteils (48) zu bewirken.

11. System (14) nach Anspruch 1, bei dem der Körper des Halters (26) eine erste Raste (50) und eine zweite Raste (52) aufweist, wobei die zweite Raste (52) axial von der ersten Raste (50) beabstandet ist, wobei der Schutz (28) einen Vorsprung (58) aufweist, der zwischen die erste (50) und die zweite Raste (52) positionierbar ist, wenn sich der Schutz (28) in der ausgefahrenen Position befindet.

12. System (14) nach einem der Ansprüche 1-11, mit einer Spritze (12), die einen Zylinder (16) mit einem offenen proximalen Ende und einem distalen Ende, an dem eine Nadel (18) angebracht ist, aufweist, wobei die Spritze (12) mit dem Halter (26) verbunden und gleitend verschiebbar in einer durch den Körper gebildeten Hülle angeordnet ist, und wobei die Spritze (12) axial in der Hülle verschiebbar ist.

13. Spritze (12) in Kombination mit einem Schutzsystem (14) nach einem der Ansprüche 1-11, wobei die Spritze (12) einen Zylinder (16) mit einem offenen proximalen Ende und einem distalen Ende, an dem eine Nadel (18) angebracht ist, aufweist, wobei die Spritze (12) mit dem Halter (26) verbunden und gleitend verschiebbar in einer durch den Körper gebildeten Hülle angeordnet ist, und wobei die Spritze (12) axial in der Hülle verschiebbar ist.

## Revendications

1. Système de protection (14) pour une seringue (12) ayant un cylindre (16) ayant une extrémité proximale ouverte et une extrémité distale ayant une aiguille (18) qui y est fixée, ledit système (14) comprenant :
un support (26) comprenant un corps allongé (34), un boîtier allongé défini par ledit corps, et une extrémité proximale et une extrémité distale ouvertes, la seringue pour être tenue dans ledit support et pour pouvoir y être mobile ;
une protection allongée (28) raccordée au dit support (26) et ayant une partie d'extrémité distale, ladite protection (28) pouvant être déplacée axialement relativement au dit support (26), ladite protection (28) étant positionnée au moins en partie dans ledit boîtier et pouvant coulisser axialement dans ledit boîtier depuis une position rétractée, dans laquelle, lors de l'utilisation, l'aiguille (18) n'est pas contenue dans ladite protection (28), vers une position étendue dans laquelle, lors de l'utilisation, l'aiguille (18) est contenue dans ladite protection (28) ;
un élément de raccord (32) ayant une extrémité proximale pour couplage à l'extrémité proximale ouverte du cylindre (16) de la seringue, et une extrémité distale ;
un membre de butée (48) placé sur ledit support (26) et pouvant être engagé avec ladite protection (28), ledit membre de butée (48) étant positionné de manière à maintenir ladite protection (28) en position rétractée et à libérer ladite protection (28) lors d'un déplacement axial suffisant de ladite protection (28) ; et
un ressort (30) couplé à ladite protection (28) et au dit élément de raccord (32) et poussant ladite protection (28) vers une position étendue, ledit ressort (30) incluant une première extrémité et une seconde extrémité butant contre ledit élément de raccord (32),
**caractérisé en ce que**
ledit élément de raccord (32) est positionné dans ladite protection (28) et est destiné à raccorder ladite protection (28) dans le cylindre (16) de la seringue,
ladite extrémité distale dudit élément de raccord (32) est couplé à ladite partie d'extrémité distale de ladite protection (28), et
ladite première extrémité dudit ressort (30) bute contre ladite partie d'extrémité distale de ladite protection (28).

2. Système (14) comme décrit dans la revendication 1 dans lequel ledit moyen de couplage comprend un raccord terminal allongé (32) couplé de manière coulissante au dit support (26), ledit ressort (30) étant couplé à ladite protection (28) par ledit raccord terminal (32).

3. Système (14) comme décrit dans la revendication 2 dans lequel ladite protection (28) est montée de manière coulissante sur ledit raccord terminal (32).

4. Système (14) comme décrit dans la revendication 3 dans lequel ladite protection (28) inclut un dispositif de retenue du ressort (46) près de son ouverture distale, ledit ressort (30) ayant une extrémité retenue par ledit dispositif de retenue du ressort (46).

5. Système (14) comme décrit dans la revendication 1 dans lequel ledit moyen de couplage comprend un raccord terminal allongé (32) monté de manière coulissante au dit support (26), ladite protection (28) étant montée de manière coulissante sur ledit raccord terminal (32), ledit ressort (30) ayant une première extrémité engageant ledit raccord terminal (32) et une seconde extrémité engageant ladite protection (28).

6. Système (14) comme décrit dans la revendication 5 dans lequel ladite première extrémité dudit ressort (30) s'étend sur une extrémité distale dudit raccord terminal (32).

7. Système (14) comme décrit dans la revendication 6 dans lequel ladite protection (28) inclut un dispositif de retenue de ressort (46) proche de son ouverture distale, ladite seconde extrémité dudit ressort (30) étant retenue par ledit dispositif de retenue de ressort (46).

8. Système (14) comme décrit dans la revendication 4 dans lequel ledit dispositif de retenue de ressort (46) définit une extrémité de ladite protection (28).

9. Système (14) comme décrit dans la revendication 8 dans lequel ledit raccord terminal (32) inclut un épaulement (33) engageant ladite seconde extrémité dudit ressort.

10. Système (14) comme décrit dans la revendication 1 incluant un dispositif de retenue à une partie d'extrémité proximale dudit support (26), ledit support (26) incluant une partie d'extrémité distale incluant un élément de butée (48) pouvant être engagé avec ladite protection (28) lorsque ladite protection (28) est dans sa position rétractée, la force dudit ressort (30) étant insuffisante pour provoquer le désengagement de ladite protection (28) et dudit membre de butée (48).

11. Système (14) comme décrit dans la revendication 1 dans lequel ledit corps dudit support (26) inclut un premier cran d'arrêt (50) et un second cran d'arrêt (52), ledit second cran d'arrêt (52) étant espacé axialement dudit premier cran d'arrêt (50), ladite protection (28) incluant une projection (58) pouvant être positionnée entre ledit premier cran d'arrêt (50) et ledit second cran d'arrêt (52) lorsque ladite protection (28) est en position étendue.

12. Système (14) comme décrit dans l'une des revendications 1 à 11, comprenant une seringue (12) incluant un cylindre (16) ayant une extrémité proximale ouverte et une extrémité distale ayant une aiguille (18) lui étant fixée, ladite seringue (12) étant couplée au dit support (26) et étant positionnée de manière coulissante dans un boîtier défini par ledit corps, ladite seringue (12) pouvant coulisser axialement dans ledit boîtier.

13. Seringue (12) en association avec un système de protection (14) selon l'une quelconque des revendications 1 à 11, ladite seringue (12) incluant un cylindre (16) ayant une extrémité proximale ouverte et une extrémité distale ayant une aiguille (18) lui étant fixée, ladite seringue (12) étant couplée au dit support (26) et étant positionnée de manière à coulisser dans un boîtier défini par ledit corps, ladite seringue (12) pouvant coulisser axialement dans ledit boîtier.
